Europäisches Patentamt

(19) **European Patent Office**

**Office européen des brevets**

(11) Numéro de publication: **0 080 406**
**B1**

(12) # FASCICULE DE BREVET EUROPEEN

(45) Date de publication du fascicule du brevet: **14.05.86**

(51) Int. Cl.⁴: **G 01 N 27/16**, G 01 N 33/00

(21) Numéro de dépôt: **82402094.5**

(22) Date de dépôt: **17.11.82**

(54) Procédé d'interrogation d'un détecteur de teneur en gaz combustible et dispositif pour sa mise en oeuvre.

(30) Priorité: **20.11.81 FR 8121733**

(43) Date de publication de la demande:
**01.06.83 Bulletin 83/22**

(45) Mention de la délivrance du brevet:
**14.05.86 Bulletin 86/20**

(84) Etats contractants désignés:
**BE DE GB IT**

(56) Documents cités:
**EP - A - 0 068 959**
**FR - A - 1 397 436**
**GB - A - 1 155 481**
**US - A - 4 028 057**

**Le dossier contient des informations techniques présentées postérieurement au dépôt de la demande et ne figurant pas dans le présent fascicule.**

(73) Titulaire: **Etablissement public dit: CHARBONNAGES DE FRANCE, 9, Avenue Percier, F-75008 Paris (FR)**

(72) Inventeur: **Boutonnat, Maurice, Route des Carrières, F-60270 Gouvieux (FR)**
Inventeur: **Rose, Gérard, 100 rue A. Briand, F-60470 Villers Saint Paul (FR)**

(74) Mandataire: **Rinuy, Santarelli, 14, avenue de la Grande Armée, F-75017 Paris (FR)**

## Description

La présente invention concerne un procédé d'interrogation d'un détecteur de teneur en gaz combustible et un dispositif pour sa mise en œuvre.

On connait différents types d'explosimètres dans lesquels la teneur d'une atmosphère en gaz combustible est mesurée par combustion catalytique sur un filament de platine, ladite combustion provoquant un échauffement et donc une variation de résistance. Celle-ci est utilisée pour provoquer le déséquilibre d'un pont de Wheatstone alimenté par une source de courant continu et dans lequel est inséré ledit filament, déséquilibre que l'on mesure finalement.

Ainsi le brevet britannique GB-A-1.155.481 décrit un dispositif de mesure de la teneur en gaz combustible d'une atmosphère et un procédé permettant d'utiliser, sans le découper, le signal de déséquilibre d'un pont de mesure, quelle que soit sa polarité, en vue d'obtenir, de façon univoque, un déclenchement ou une alarme pour toutes les valeurs de la teneur en gaz combustible supérieures à un certain seuil.

On connait également des détecteurs de teneur en gaz combustible constitués d'un filament métallique enrobé par un matériau réfractaire poreux.

Pour ces différents types de détecteurs de teneur en gaz combustible, notamment pour les explosimètres automatiques, on procède généralement par la voie d'une interrogation discontinue. Pour certains gaz, en particulier le méthane, le résultat d'une interrogation du détecteur dans une atmosphère à forte teneur en gaz (c'est-à-dire dont la teneur dépasse un certain seuil fonction de la nature dudit gaz) est normalement constitué par l'inversion du signal de mesure délivré par le détecteur. L'obtention de ce phénomène nécessite généralement un traitement spécifique des filaments.

Le problème à résoudre par la présente invention consiste en ce que l'emploi de l'inversion du signal délivré par le détecteur ne présente pas une garantie totale, surtout dans les détecteurs automatiques à interrogation discontinue, quant à la certitude d'une forte teneur en gaz combustible dans l'atmosphère considérée. Plus précisément, on a constaté que le phénomène d'inversion du signal de mesure n'est pas conservatif et que certains détecteurs, même à l'état neuf, peuvent donner des indications ambiguës à cet égard.

La présente invention vise donc à définir une solution totalement fiable au problème de sécurité exposé ci-dessus et de réaliser un procédé d'interrogation d'un détecteur de teneur en gaz combustible permettant d'indiquer sans ambiguïté si la concentration en un tel gaz combustible dans une atmosphère est ou n'est pas supérieure à un seuil prédéterminé, ainsi qu'un dispositif mettant en œuvre ce procédé.

Le procédé conforme à l'invention est un procédé d'interrogation d'un détecteur de teneur en gaz combustible d'une atmosphère, lequel détecteur comporte un pont de mesure contenant un élément catalytique dont la résistance varie en fonction de la teneur en gaz combustible de ladite atmosphère, procédé selon lequel on alimente le pont de mesure en courant continu et on détecte le signal de sortie du pont, un déséquilibrage du pont provoqué par la présence d'un gaz combustible résultant en une polarité soit positive, soit négative dudit signal de sortie, ce procédé étant caractérisé en ce que:

– dans une première étape, du temps 0 au temps $t_1$ de la séquence d'interrogation, on alimente le détecteur sous une tension $V_1$ faible et on coupe l'alimentation dès qu'un signal positif a été détecté, et
– dans une seconde étape, si le signal délivré par le détecteur est négatif ou nul jusqu'au temps $t_1$, on alimente le détecteur, du temps $t_1$ au temps $t_2$ de la séquence d'interrogation, sous une tension $V_2$ supérieure à $V_1$ puis on mesure l'amplitude du signal au temps $t_2$.

L'application du procédé selon l'invention à la détection d'une teneur élevée en gaz combustible est la suivante: si le signal devient positif durant la première étape du procédé, il y a eu oxydation donc teneur élevée et, dans ce cas, il est inutile de poursuivre la mesure en usant inutilement le filament. Si le signal demeure négatif ou nul durant la première étape du procédé, on ne peut pas conclure et il faut poursuivre la mesure sous une tension $V_2$ supérieure à $V_1$. Si le signal mesuré dans la seconde étape du procédé est négatif, il y a eu inversion et on sait que la teneur est élevée. Par contre si le signal mesuré à l'issue de la seconde étape du procédé est positif, la teneur est faible et on la mesure par l'amplitude du signal. Selon la valeur fournie par cette mesure, comparée au seuil prédéterminé de la concentration en gaz combustible, on déclenchera ou non un dispositif d'alarme.

Le choix des paramètres de fonctionnement du procédé selon l'invention, à savoir $t_1$, $t_2$, $V_1$ et $V_2$, dépend de la nature du gaz combustible considéré et répond généralement aux deux exigences de l'utilisateur exprimées comme suit:

– la durée totale $t_2$ de l'interrogation n'est pas trop grande, et
– le procédé ne conduit jamais à des indications ambiguës.

Par gaz combustible au sens de l'invention on entend par exemple les hydrocarbures saturés ayant de 1 à 4 atomes de carbone d'une part et l'éthylène d'autre part.

Le demandeur a trouvé que, pour satisfaire les exigences ci-dessus dans le cas du méthane, il est préférable, dans le cas de l'utilisation de détecteurs de type courant, de choisir les paramètres comme indiqué ci-après:

– $t_1$ est compris entre 0,5 et 0,7 seconde,

- $t_2$ est compris entre 1,9 et 2 secondes,
- $V_1$ est compris entre 0,9 et 1,0 volt,
- $V_2$ est compris entre 1,2 et 1,4 volt.

Dans ces conditions de fonctionnement, le procédé selon l'invention assure une garantie totale quant à la fiabilité de détermination d'une teneur en gaz combustible dans une atmosphère, quelle que soit cette teneur comprise entre 0 et 100%.

Le procédé selon l'invention procure un avantage supplémentaire par rapport au procédé d'interrogation à une seule tension élevée. En effet dans ce dernier procédé, on a observé que des mesures répétées dans des teneurs élevées en gaz combustible, par exemple dans des teneurs en méthane supérieures à 30%, modifiaient la forme de la courbe du signal par suite de l'apparition d'un phénomène de cracking provoquant un dépôt de carbone sur le filament détecteur. Le fait de couper l'alimentation du détecteur dès qu'un signal positif a été détecté dans la première étape du procédé selon l'invention supprime bien évidemment ce phénomène parasite.

Enfin le procédé selon l'invention est d'un emploi très général et ne dépend pas de la nature du filament. Il est applicable en liaison avec tout pont de mesure fournissant un signal de déséquilibre polarisé et d'amplitude quelconque, qu'il présente ou non le phénomène d'inversion.

Le procédé selon l'invention est particulièrement avantageux dans son application à la détection de teneurs en méthane dans les mines de charbon.

Le dispositif de détection de la teneur en gaz combustible d'une atmosphère pour la mise en œuvre du procédé décrit ci-dessus comprend un détecteur en forme de pont de mesure contenant un élément catalytique dont la résistance varie en fonction de la teneur en gaz combustible dans ladite atmosphère, une source d'alimentation fournissant une tension $V^+$ et alimentant le pont de mesure en courant continu, et un amplificateur du signal de sortie dudit pont de mesure, et est caractérisé en ce qu'il comprend en outre un circuit d'abaissement de la tension $V^+$ relié d'une part à la masse par un premier interrupteur et d'autre part au pont de mesure par un second interrupteur et délivrant au pont de mesure l'une ou l'autre de deux tensions $V_1$ et $V_2$ à partir de la source d'alimentation selon la position du second interrupteur, un discriminateur asservissant le premier interrupteur au signe et/ou à l'amplitude du signal S délivré par l'amplificateur, et une base de temps capable d'initialiser la séquence d'interrogation, agissant sur le deuxième interrupteur.

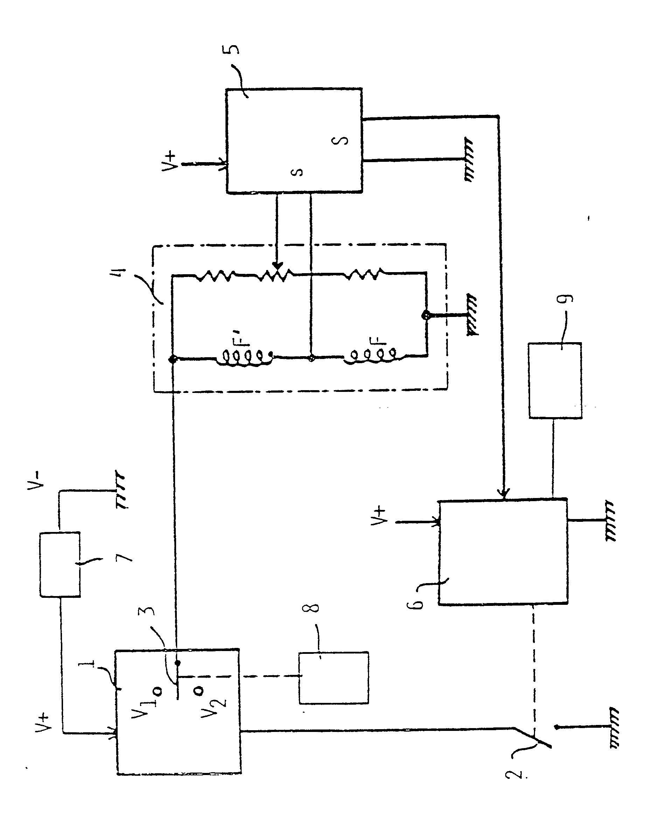

La figure unique illustre un mode de réalisation du dispositif selon l'invention.

Le détecteur (4) est par exemple constitué d'un pont de Wheatstone dont les quatre branches sont formées respectivement par le filament détecteur F, le filament compensateur F′, une première résistance et une seconde résistance en série avec un potentiomètre de réglage du zéro, réglage effectué en absence de toute trace de gaz combustible. Lors de la détection de la présence de gaz combustible dans une atmosphère, le pont soumis à la tension d'interrogation engendre un signal de déséquilibre (s) qui est généralement une tension.

L'amplificateur (5) est un amplificateur de courant continu dont l'alimentation est assurée par une source de tension positive. Il transforme la valeur du signal (s) en une valeur plus élevée (S).

Le circuit d'alimentation (1) comprend des éléments capables d'abaisser la tension fournie par la source positive aux deux valeurs choisies $V_1$ et $V_2$, $V_1$ étant inférieure à $V_2$. La source de tension positive peut être constituée par une ou des piles, une batterie ou un transformateur-redresseur alimenté par le secteur et comprenant de préférence un circuit stabilisateur. Les interrupteurs 2 et 3 peuvent être de tout type connu. Avantageusement ils sont du type électronique.

Le discriminateur (6) est constitué de circuits effectuant des opérations logiques; il asservit l'interrupteur (2) au signe et/ou à l'amplitude du signal S. La base de temps (8) initialisant la séquence d'interrogation est commandée soit manuellement soit électroniquement.

Le dispositif selon l'invention peut avantageusement être associé à un système d'alarme (9) commandé par le signe et/ou l'amplitude du signal S et relié soit à l'amplificateur (5) soit, comme représenté sur la figure en annexe, au discriminateur (6). L'alarme peut être sonore et/ou visuelle. Enfin le dispositif selon l'invention peut en outre être associé à un système de traitement du signal S, relié à l'amplificateur (5) et capable de convertir l'amplitude dudit signal en la teneur en gaz combustible.

Le dispositif selon l'invention fonctionne de la façon suivante. Au temps $t = 0$ l'initialisation de la séquence d'interrogation provoque la fermeture des interrupteurs (2) et (3) précédemment ouverts. L'interrupteur (3) se ferme sur la position $V_1$. Au temps $t_1$, si le signal S est positif, le discriminateur (6) provoque l'ouverture de l'interrupteur (2), et le système d'alarme associé est déclenché. Au temps $t_1$, si le signal S est négatif ou nul, le discriminateur maintient la fermeture de l'interrupteur (2) et la base de temps (8) bascule l'interrupteur (3) sur la position $V_2$ déclenchant ainsi la seconde phase de la séquence d'interrogation. Au temps $t_2$, qui marque la fin de la séquence d'interrogation, si le signal S demeure négatif ou nul le système d'alarme associé est déclenché; si le signal est positif le système d'alarme associé ne sera déclenché que si la valeur du signal S est supérieure à celle correspondant à un seuil prédéterminé de teneur en gaz combustible. Enfin, au temps $t_2$, le discriminateur (6) provoque l'ouverture de l'interrupteur (2) et la base de temps (8) celle de l'interrupteur (3). Dans tous les cas de fonctionnement décrits ci-dessus le système de traitement du signal S associé au dispositif selon l'invention permet de connaître la teneur en gaz combustible détecté.

## Revendications

1. Procédé d'interrogation d'un détecteur de teneur en gaz combustible d'une atmosphère, lequel détecteur comporte un pont de mesure (4) contenant un élément catalytique (F) dont la résistance varie en fonction de la teneur en gaz combustible de ladite atmosphère, procédé selon lequel on alimente le pont de mesure en courant continu et on détecte le signal de sortie du pont, un déséquilibrage du pont provoqué par la présence d'un gaz combustible résultant en une polarité soit positive, soit négative dudit signal de sortie, procédé caractérisé en ce que:

– dans une première étape, du temps 0 au temps $t_1$ de la séquence d'interrogation, on alimente le détecteur sous une tension $V_1$ faible et on coupe l'alimentation dès qu'un signal positif a été détecté, et
– dans une seconde étape, si le signal délivré par le détecteur est négatif ou nul jusqu'au temps $t_1$, on alimente le détecteur, du temps $t_1$ au temps $t_2$ de la séquence d'interrogation, sous une tension $V_2$ supérieure à $V_1$ puis on mesure l'amplitude du signal au temps $t_2$.

2. Procédé d'interrogation selon la revendication 1, caractérisé en ce que $t_1$ est compris entre 0,5 et 0,7 secondes, $t_2$ est compris entre 1,9 et 2 secondes, $V_1$ est compris entre 0,9 et 1,0 volt et $V_2$ est compris entre 1, 2 et 1,4 volt.

3. Application du procédé selon l'une des revendications 1 et 2 à la détection de teneur en méthane.

4. Dispositif de détection de la teneur en gaz combustible d'une atmosphère, mettant en œuvre le procédé selon la revendication 1 et comprenant un détecteur en forme de pont de mesure (4) contenant un élément catalytique (F) dont la résistance varie en fonction de la teneur en gaz combustible dans ladite atmosphère, une source d'alimentation (7) fournissant une tension $V^+$ et alimentant le pont de mesure en courant continu, et un amplificateur (5) du signal de sortie dudit pont de mesure, le dispositif étant caractérisé en ce qu'il comprend en outre un circuit (1) d'abaissement de la tension $V^+$ relié d'une part à la masse par un premier interrupteur (2) et d'autre part au pont de mesure (4) par un second interrupteur (3) et délivrant au pont de mesure l'une ou l'autre de deux tensions $V_1$ et $V_2$ à partir de la source d'alimentation (7) selon la position du second interrupteur, un discriminateur (6) asservissant le premier interrupteur (2) au signe et/ou à l'amplitude du signal S délivré par l'amplificateur (5), et une base de temps (8) capable d'initialiser la séquence d'interrogation, agissant sur le deuxième interrupteur (3).

5. Dispositif selon la revendication 4 caractérisé en ce qu'il comprend en outre un système d'alarme relié à l'amplificateur (5) et commandé par le signe et/ou l'amplitude du signal S.

6. Dispositif selon la revendication 4 caractérisé en ce qu'il comprend en outre un système d'alarme (9) relié au discriminateur (6) et commandé par le signe et/ou l'amplitude du signal S.

7. Dispositif selon l'une des revendications 4 à 6 caractérisé en ce qu'il comprend en outre un système de traitement du signal S relié à l'amplificateur (5) et capable de convertir l'amplitude dudit signal en la teneur en gaz combustible.

## Patentansprüche

1. Abfrageverfahren für einen Detektor für den Gehalt eines brennbaren Gases in einer Atmosphäre, wobei der Detektor eine Messbrücke (4) mit einem katalytischen Element (F) besitzt, dessen Widerstand sich in Abhängigkeit von dem Gehalt des brennbaren Gases in der Atmosphäre ändert, nach dem Verfahren die Messbrücke mit Gleichstrom gespeist und das Ausgangssignal der Brücke erfasst wird und ein durch die Anwesenheit eines brennbaren Gases hervorgerufenes Ungleichgewicht der Brücke eine positive oder negative Polarität des Ausgangssignals ergibt, dadurch gekennzeichnet, dass:

– während eines ersten Abschnittes von der Zeit 0 bis zur Zeit $t_1$ der Abfragefolge der Detektor mit einer niederen Spannung $V_1$ gespeist und die Speisung desselben abgebrochen wird, falls ein positives Signal erfasst wird, und
– während eines zweiten Abschnittes, wenn das durch den Detektor gelieferte Signal bis zur Zeit $t_1$ negativ oder 0 ist, der Detektor von der Zeit $t_1$ bis zur Zeit $t_2$ der Abfragefolge mit einer Spannung $V_2$ höher als $V_1$ gespeist wird bis zur Messung der Amplitude des Signals zur Zeit $t_2$.

2. Abfrageverfahren nach Anspruch 1, dadurch gekennzeichnet, dass $t_1$ zwischen 0,5 und 0,7 s, $t_2$ zwischen 1,9 und 2 s, $V_1$ zwischen 0,9 und 1,0 V und $V_2$ zwischen 1,2 und 1,4 V beträgt.

3. Anwendung des Verfahrens nach einem der Ansprüche 1 und 2 bei der Erfassung des Gehaltes von Methan.

4. Vorrichtung zum Erfassen des Gehaltes an brennbarem Gas in einer Atmosphäre zur Durchführung des Verfahrens nach Anspruch 1 und mit einem Detektor in Form der Messbrücke (4) mit einem katalytischen Element (F), dessen Widerstand sich in Abhängigkeit von dem Gehalt an brennbarem Gas in der Atmosphäre ändert, einer eine Spannung $V^+$ liefernden Versorgungsquelle, die die Messbrücke mit Gleichstrom speist und einem Verstärker (5) für das Ausgangssignal der Messbrücke, dadurch gekennzeichnet, dass die Vorrichtung unter anderem einen Abschwächkreis (1) für die Spannung $V^+$ enthält, der einerseits über einen ersten Schalter (2) mit Masse und andererseits über einen zweiten Schalter (3) mit der Messbrücke (4) verbunden ist und der Messbrücke die eine oder die andere der beiden Spannungen $V_1$ und $V_2$ auf Grundlage der Versorgungsquelle (7) gemäss der Stellung des zweiten Schalters liefert, einen den ersten Schalter (2) gemäss dem Vorzeichen und/oder der Amplitude des durch den Verstärker (5) gelieferten Signals S steuernden Diskriminator (6) und eine auf den zweiten Schalter (3) einwirkende, zur Einleitung der Abfragefolge fähige Zeitbasis (8).

5. Vorrichtung nach Anspruch 4, dadurch gekennzeichnet, dass sie unter anderem ein mit dem Verstärker (5) verbundenes und durch das Vorzeichen und/oder die Amplitude des Signals S gesteuertes Alarmsystem enthält.

6. Vorrichtung nach Anspruch 4, dadurch gekennzeichnet, dass sie unter anderem ein mit dem Diskriminator (6) verbundenes und durch das Vorzeichen und/oder die Amplitude des Signals S gesteuertes Alarmsystem (9) enthält.

7. Vorrichtung nach einem der Ansprüche 4 bis 6, dadurch gekennzeichnet, dass sie unter anderem ein an dem Verstärker (5) angeschlossenes Verarbeitungssystem für das Signal S zum Wandeln der Amplitude des Signals in den Gehalt an brennbarem Gas enthält.

## Claims

1. A method of interrogating a sensor for detecting combustible gas contents in an atmosphere, such sensor comprising a measurement bridge (4) containing a catalytic element (F) therein, the resistance of which varies as a function of the combustible gas contents in said atmosphere, the method being such that the measurement bridge is supplied with direct current and the output signal from the bridge is detected, imbalance in the bridge caused by the presence of a combustible gas resulting into a polarity either positive or negative of said output signal, the method being characterized in that:

– in a first step, from the time 0 to the time $t_1$ of the interrogation sequence, the sensor is supplied under a little voltage $V_1$ and the power supply is switched off as soon as a positive signal is detected, and
– in a second step, if the signal produced by the sensor is negative, or 0 up to the time $t_1$, the sensor is fed from time $t_1$ to time $t_2$ of the interrogation sequence under a voltage $V_2$ higher than $V_1$ and then, the amplitude of the signal at time $t_2$ is measured.

2. An interrogation method according to claim 1, characterized in that $t_1$ is comprised between 0.5 and 0.7 seconds, $t_2$ is comprised between 1.9 and 2 seconds, $V_1$ is comprised between 0.9 and 1.0 volt and $V_2$ is comprised between 1.2 and 1.4 volts.

3. An application of the method according to one of claims 1 and 2, to the detection of methane contents.

4. A device for detecting the combustible gas contents of an atmosphere and for carrying out the method according to claim 1, and comprising a sensor in form of a measurement bridge (4) containing a catalytic element (F) the resistance of which varies as a function of the combustible gas contents in said atmosphere, a power supply (7) providing a voltage $V^+$ and supplying direct current to the measurement bridge, and an amplifier (5) of the output signal from the measurement bridge, the device being characterized in that it comprises moreover a voltage $V^+$ reduction circuit (1) connected to the ground through a first switch (2) on the one hand, and on the other hand, to the measurement bridge (4) through a second switch (3) and supplying the measurement bridge with one or the other of two voltages $V_1$ and $V_2$ from the power supply (7) depending of the position of the second switch, a discriminator (6) servo-controlling the first switch (2), to the sign and/or the amplitude of the signal (S) supplied by the amplifier (5), and a time base (8) capable of initiating the interrogation sequence acting upon the second switch (3).

5. A device according to claim 4, characterized in that it also comprises an alarm system connected to the amplifier (5) and controlled by the sign and/or amplitude of the signal (S).

6. A device according to claim 4, characterized in that it also comprises an alarm signal (9) connected to the discriminator (6) and controlled by the sign and/or the amplitude of signal (S).

7. A device according to one of claims 4 to 6, characterized in that it also comprises a system for processing the signal (S) connected to the amplifier (5) and suitable for converting the amplitude of said signal to the combustible gas contents.